# EUROPÄISCHE PATENTSCHRIFT

(11) **EP 1 113 809 B1**
(45) Veröffentlichungstag und Bekanntmachung des Hinweises auf die Patenterteilung: **06.04.2005**
(21) Anmeldenummer: 99969031.6
(22) Anmeldetag: 09.09.1999
(51) Int. Cl.: A61K 38/12, A61P 7/00, A61P 9/00, A61P 19/00, A61P 27/00, A61P 31/00, A61P 35/00

(54) **PHARMAZEUTISCHE ZUBEREITUNG ENTHALTEND EIN CYCLO-PEPTIDE UND EIN CHEMOTHERAPEUTIKUM ODER EINEN ANGIOGENESEINHIBITOR**
PHARMACEUTICAL PREPARATION CONTAINING A CYCLOPEPTIDE AND A CHEMOTHERAPEUTIC AGENT OR AN ANGIOGENESIS INHIBITOR
PREPARATION PHARMACEUTIQUE CONTENANT UN CYCLOPEPTIDE ET UN AGENT CHIMIOTHERAPEUTIQUE OU UN INHIBITEUR D'ANGIOGENESE

(30) Priorität: 16.09.1998 DE 19842415
(43) Veröffentlichungstag der Anmeldung: 11.07.2001
(60) Teilanmeldung: 04006076.6
(73) Patentinhaber: MERCK PATENT GmbH, 64293 Darmstadt (DE)
(72) Erfinder: JONCZYK, Alfred, D-64295 Darmstadt (DE); PERSCHL, Astrid, D-64297 Darmstadt (DE); GOODMAN, Simon, D-64286 Darmstadt (DE); RÖSENER, Sigrid, D-63110 Rodgau (DE); HAUNSCHILD, Jutta, D-85540 Haar (DE)
(86) Internationale Anmeldenummer: PCT/EP1999/006654
(87) Internationale Veröffentlichungsnummer: WO 2000/015244

(56) Entgegenhaltungen:
- WO-A-97/41844
- WO-A-97/45447
- WO-A-98/14192
- WO-A-98/31359
- DE-A- 19 534 177
- LODE H N ET AL: "Synergy between an antiangiogenic integrin alphav antagonist and an antibody-cytokine fusion protein eradicates spontaneous tumor metastases." PROCEEDINGS OF THE NATIONAL ACADEMY OF SCIENCES OF THE UNITED STATES OF AMERICA, (1999 FEB 16) 96 (4) 1591-6. , XP002134006

## Beschreibung

Gegenstand der Erfindung ist eine neue pharmazeutische Zubereitung enthaltend Cyclo-(Arg-Gly-Asp-D-Phe-NMe-Val) und/oder eines seiner physiologisch unbedenklichen Salze und mindestens ein Chemotherapeutikum und/oder eines seiner physiologisch unbedenklichen Salze, das dadurch gekennzeichnet ist, dass das Chemotherapeutikum Gemcitabine eingesetzt wird.

Diese neue Zubereitung kann zur Bekämpfung von Tumoren, verwendet werden.

Der Erfindung lag die Aufgabe zugrunde, neue Arzneimittel in Form von pharmazeutischen Zubereitungen zur Verfügung zu stellen, die bessere Eigenschaften besitzen als bekannte, für die gleichen Zwecke verwendbare Arzneimittel.

Diese Aufgabe wurde durch das Auffinden der neuen Zubereitung gelöst.

Cyclo-(Arg-Gly-Asp-D-Phe-NMe-Val) ist aus EP 0 770 622 bekannt und wirkt vor allem als Integrin-Inhibitor, wobei es insbesondere die Wechselwirkungen der α_{V}-, β₃- oder β₅-Integrin-Rezeptoren mit Liganden hemmt, wie z. B. die Bindung von Fibrinogen an den α_{V}β₃-Integrinrezeptor. Besondere Wirksamkeit zeigt die Verbindung im Fall der Integrine α_{V}β₃, α_{V}β₅, α_{IIb}β₃ sowie α_{V}β₁, α_{V}β₆ und α_{V}β₈.
Diese Wirkung kann z.B. nach der Methode nachgewiesen werden, die von J.W. Smith et al. in J. Biol. Chem. 265, 12267-12271 (1990) beschrieben wird.

Die vorliegende Erfindung ist in bezug auf EP 0 770 622 als Auswahlerfindung anzusehen.

In der WO 98/14192 sind pharmazeutische Präparate genannt, die Kombinationen von nicht-peptidischen Vitronektinrezeptorantagonisten mit Chemotherapeutika enthalten.
Der Effekt einer Antiangiogenesetherapie kombiniert mit einer Chemotherapie ist von J. Folkman in Nature Medicine 1, 27-30 (1995) beschrieben.

Die Wirksamkeit von Cyclo-(Arg-Gly-Asp-D-Phe-NMe-Val) in Kombination mit einem Chemotherapeutikum kann am Lewis-Lungenkarzinomsystem gezeigt werden. Das Lewis-Lungenkarzinom wird durch konventionelle Chemotherapeutika nur unzureichend beeinflußt (Y. Kakeji und B.A. Teicher, Invest. New Drugs 15: 39-48 (1997)).
Das Verfahren zur Verzögerung des Tumorwachstums wird analog Kakeji durchgeführt (F. Mitjans et al., J. Cell Sci. 108: 2825-2838 (1995)).

Gegenstand der Erfindung ist daher eine pharmazeutische Zubereitung enthaltend Cyclo-(Arg-Gly-Asp-D-Phe-NMe-Val) und/oder eines seiner physiologisch unbedenklichen Salze und mindestens ein Chemotherapeutikum und/oder eines seiner physiologisch unbedenklichen Salze, die dadurch gekennzeichnet ist, dass das Chemotherapeutikum Gemcitabine eingesetzt wird.

Gegenstand der Erfindung ist insbesondere eine pharmazeutische Zubereitung wie beschrieben, dadurch gekennzeichnet, daß zusätzlich ein Chemotherapeutikum aus einer Gruppe bestehend aus
a) alkylierenden Agenzien,
b) Antibiotika,
c) Antimetaboliten,
d) Biologika und Immunmodulatoren,
e) Hormonen und deren Antagonisten,
f) Senfgasderivaten,
g) Alkaloiden,
h) Inhibitoren der Matrix-Metallo-Proteinasen (MMP-Inhibitoren),
i) Protein-Kinase-Inhibitoren,
k) anderen
eingesetzt wird.

Als alkylierende Agenzien sind z.B. Busulfan, Carboplatin, Carmustine, Cisplatin, Cyclophosphamid, Dacarbazine, Ifosfamide oder Lomustine bevorzugt.
Als Antibiotika sind z.B. Bleomycin, Doxorubicin (Adriamycin), Idarubicin oder Plicamycin bevorzugt.
Als Antimetaboliten sind z.B. Sulfonamide oder Folsäureantagonisten bevorzugt, wie z.B. auch 5-Fluorouracil (5-FU), Mercaptopurin, Methotrexat oder Thioguanin oder 5-FU mit Calciumfolinat (Leucovorin).
Als Biologika und Immunmodulatoren sind z.B. Interferon a2A, Interleukin 2 oder Levamisole bevorzugt.
Als Hormone und deren Antagonisten sind z.B. Flutamide, Goserelin, Mitotane oder Tamoxifen bevorzugt.
Als Senfgasderivate sind z.B. Melphalan, Carmustine oder Stickstofflost bevorzugt.
Als Alkaloide sind z.B. die Taxane wie Docetaxel oder Paclitaxel bevorzugt, ferner Etoposide, Vinblastine oder Vinovelbine.
Unter anderen Chemotherapeutika werden solche verstanden, die nicht den vorstehenden Gruppen zugeordnet werden können, wie z.B.
Altretamine, Cladribine, Gemcitabine, Leucovorin, Levamisole, Pentostatin oder Irinotecan.

Unter Inhibitoren der Matrix-Metallo-Proteinasen (MMP-Inhibitoren), die z.B. auch von M.Wittaker et al. in *Current Opinion in Drug Discovery & Development 1, 157-164* (*1998*) beschrieben sind, sind die nachstehenden Verbindungen bevorzugt, z.B. Baltimastat (BB-94), Marimastat (BB-2516), BB-3644, Ilomastat, Metastat, AG-3340, BAY-12-9566, AE-941/Neovastat, CGS-27023A, RS-113456, RS-130830, Ro-32-3555, Ro-31-9790, CT-1746, CT-1418, D-1927, D-2163.

Protein-Kinase-Inhibitoren sind z.B. durch G.McMahon et al. in *Current Opinion in Drug Discovery & Development 1, 131-146 (1998)* und von L.M. Strawn et al. in *Exp. Opin. Invest. Drugs 7, 553-573 (1998)* beschrieben. In den erfindungsgemäßen Zubereitungen sind speziell die nachstehenden Rezeptor-Tyrosin-Kinase-Inhibitoren bevorzugt:
CGP 79787, SU-101 (HWA 486, Leflunomide, Arava), SU-5416, SU-5271 (PD-153035), PD-173074, SU-6668, ZD-1839, CP-358774.

In den erfindungsgemäßen Zubereitungen sind auch sogenannte Prodrug-Derivate der Angiogeneseinhibitoren und/oder der Chemotherapeutika eingeschlossen, d. h. mit z. B. Alkyl- oder Acylgruppen, Zuckern oder Oligopeptiden abgewandelte Verbindungen, die im Organismus rasch zu den wirksamen erfindungsgemäßen Verbindungen gespalten werden. Zu den Prodrug-Derivaten gehört z.B. auch das Chemotherapeutikum Capecitabine, welches das Prodrug von 5-FU darstellt, wie dies z. B. in Inpharma No. 1142, 13-14 (1998) beschrieben ist.

Weiterer Gegenstand der Erfindung ist eine pharmazeutische Zubereitung wie beschrieben, dadurch gekennzeichnet, daß ein Chemotherapeutikum aus einer Gruppe bestehend aus Docetaxel, Paclitaxel, Carboplatin, Cisplatin, 5-FU und Calciumfolinat, lrinotecan, Cyclophosphamid, Carmustine, Doxorubicin, Vinorelbine oder Goserelin eingesetzt wird.

Die Verwendung von Gemcitabine bei der Tumorbehandlung ist z.B. von B.J. Braakhuis et al. in Semin-Oncol. 1995 Aug; 22(4 Suppl. 11): 42-6 oder von R.M. Mohammed et al. in Pancreas 1998 Jan; 16 (1): 19-25 beschrieben.

Gegenstand der Erfindung ist eine pharmazeutische Zubereitung enthaltend Cyclo-(Arg-Gly-Asp-D-Phe-NMe-Val) und/oder eines seiner physiologisch unbedenklichen Salze und Gemcitabine und/oder eines seiner physiologisch unbedenklichen Salze.

Gegenstand der Erfindung ist weiterhin eine pharmazeutische Zubereitung wie vorstehend beschrieben, enthaltend mindestens einen Angiogenese-inhibitor und/oder eines seiner physiologisch unbedenklichen Salze.

Bevorzugte Angiogeneseinhibitoren sind z.B. in Tabelle 1 in WO 9741844 beschrieben.

Insbesondere bevorzugt sind α_{V}β₃- und α_{V}β₅-Integrininhibitoren, beispielsweise die in der EP 0 770 622 genannten Verbindungen.

Die neue pharmazeutische Zubereitung kann hergestellt werden, indem man Cyclo-(Arg-Gly-Asp-D-Phe-NMe-Val) und/oder eines seiner physiologisch unbedenklichen Salze und mindestens ein Chemotherapeutikum und/oder eines seiner physiologisch unbedenklichen Salze, wobei das Chemotherapeutikum Gemcitabine eingesetzt wird, und gegebenenfalls einen Angiogeneseinhibitor und/oder eines seiner physiologisch unbedenklichen Salze, zusammen mit mindestens einem festen, flüssigen oder halbflüssigen Träger- oder Hilfsstoff in eine geeignete Dosierungsform bringt. Die so erhaltenen Zubereitungen können als Arzneimittel in der Human- oder Veterinärmedizin, insbesondere bei der Behandlung von Tumoren, eingesetzt werden. Als Trägersubstanzen kommen organische oder anorganische Stoffe in Frage, die sich für die enterale (z.B. orale, sublinguale oder rektale), parenterale oder topische (z.B. transdermale) Applikation eignen und mit den Verbindungen nicht reagieren, beispielsweise Wasser, pflanzliche Öle, Benzylalkohole, Polyethylenglykole, Glycerinacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässrige Lösungen, ferner Suspensionen, Emulsionen oder Implantate, für die topische Anwendung Salben, Cremes oder Pflaster. Die Wirkstoffe können auch lyophilisiert und die erhaltenen Lyophilisate z.B. zur Herstellung von Injektionspräparaten verwendet werden. Die Zubereitungen können sterilisiert sein und /oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können auch weitere Wirkstoffe enthalten, z.B. andere blutdrucksenkende oder diuretisch wirkende Stoffe, aber auch Vitamine und/oder Mineralsalze, insbesondere solche, die Stoffwechselvorgänge begünstigen.

Die erfindungsgemäßen Zubereitungen werden bei der Bekämpfung von Tumoren verwendet. In der Regel werden sie zur Tumorbekämpfung, also zur Inhibierung des Tumorwachstums oder von Tumormetastasen eingesetzt.

Verwendet werden kann Cyclo-(Arg-Gly-Asp-D-Phe-NMe-Val) in solchen Kombinationspräparaten, die zur Bekämpfung von Krankheiten eingesetzt werden, bei der α_{V}-Integrine, insbesondere α_{V}β₃ und α_{V}β₅, eine Rolle spielen und dabei deren Hemmung Teil der Therapie ist.

Verwendet werden kann Cyclo-(Arg-Gly-Asp-D-Phe-NMe-Val) in der Tumortherapie, auch in Kombination mit einem anderen Angiogenese-inhibitor,
a) bei der chirurgischen Entfernung eines Tumors,
b) bei der Radiotherapie,
c) bei der photodynamischen Therapie,
d) zusammen mit monoklonalen Antikörpern gegen tumorselektive Epitope,
e) zusammen mit Fusionsproteinen,
f) zusammen mit Peptid-Vaccinen und
g) bei der Gentherapie.

Die Dosierungen von Cyclo-(Arg-Gly-Asp-D-Phe-NMe-Val) bzw. dessen Salzen als auch der Chemotherapeutika und gegebenenfalls der Angiogeneseinhibitoren liegen vorzugsweise zwischen etwa 0,1 und 100 mg, insbesondere zwischen 0,2 und 20 mg, ganz besonders zwischen 0,1 und 10 mg pro Dosierungseinheit. Die tägliche Dosierung liegt vorzugsweise zwischen etwa 0,001 und 1, insbesondere zwischen 0,002 und 0,2 mg/kg Körpergewicht.
Während einer Chemotherapie kann das Peptid beispielsweise auch in einer Dosierung von 1-10 mg/kg 2x pro Woche gegeben werden. Die Chemotherapeutika können z.B. auch in einer Dosierung von 1-10 mg/kg einmal pro Woche bis alle 3-4 Wochen verabreicht werden. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung ist daher auch die Verwendung der beschriebenen pharmazeutischen Zubereitungen zur Herstellung eines Arzneimittels zur Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen.

Gegenstand der Erfindung ist auch die Verwendung von Cyclo-(Arg-Gly-Asp-D-Phe-NMe-Val) und/oder einem seiner physiologisch unbedenklichen Salze nacheinander oder in physikalischer Kombination mit Gemcitabine und/oder einem seiner physiologisch unbedenklichen Salze und gegebenenfalls einem Angiogeneseinhibitor und/oder einem seiner physiologisch unbedenklichen Salze zur Herstellung eines Arzneimittels zur Bekämpfung von Tumoren.

Gegenstand der Erfindung ist insbesondere die Verwendung von Cyclo-(Arg-Gly-Asp-D-Phe-NMe-Val) und/oder einem seiner physiologisch unbedenklichen Salze nacheinander oder in physikalischer Kombination mit Gemcitabine und/oder einem seiner physiologisch unbedenklichen Salze zur Herstellung eines Arzneimittels zur Bekämpfung von Tumoren.

Gegenstand der Erfindung ist ferner die Verwendung von Cyclo-(Arg-Gly-Asp-D-Phe-NMe-Val) und/oder einem seiner physiologisch unbedenklichen Salze nacheinander oder in physikalischer Kombination mit Gemcitabine und/oder einem seiner physiologisch unbedenklichen Salze zur Bekämpfung von Tumoren.

Die Bestandteile der neuen pharmazeutischen Zubereitung werden vorzugsweise kombiniert verabreicht. Sie können aber auch einzeln gleichzeitig oder aufeinanderfolgend verabreicht werden.

Gegenstand der Erfindung ist auch ein Set (Kit), bestehend aus getrennten Packungen von
(a) einer wirksamen Menge an Cyclo-(Arg-Gly-Asp-D-Phe-NMe-Val) und
(b) einer wirksamen Menge des Chemotherapeutikums Gemcitabine.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an Cyclo-(Arg-Gly-Asp-D-Phe-NMe-Val) und des Chemotherapeutikums gelöst oder in lyophylisierter Form vorliegt.

Aus der WO 9814192 sind verschiedene biologische Tests bekannt, die geeignet sind, um die Konzentration der Verbindungen zu bestimmen, die einen pharmakologischen Effekt hervorrufen.

Cyclo-(Arg-Gly-Asp-D-Phe-NMe-Val), das Chemotherapeutikum oder der Angiogeneseinhibitor kann, bei Vorliegen einer Säurefunktion, mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann eine Säure mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.

Andererseits kann eine basische Funktion in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung ist weiterhin eine pharmazeutische Zubereitung wie vorstehend beschrieben, enthaltend einen α_{V}β₃- und/oder einen α_{V}β₅-Integrininhibitor und/oder eines seiner physiologisch unbedenklichen Salze und mindestens einen MMP-Inhibitor und/oder eines seiner physiologisch unbedenklichen Salze sowie eine pharmazeutische Zubereitung enthaltend einen α_{V}β₃- und/oder einen α_{V}β₅-Integrininhibitor und/oder eines seiner physiologisch unbedenklichen Salze und mindestens einen Tyrosin-Kinase-Inhibitor und/oder eines seiner physiologisch unbedenklichen Salze.

Bevorzugte α_{V}β₃- und α_{V}β₅-Integrininhibitoren sind z.B. in der EP 0 770 622, EP 0 710 657, EP 0 820 988, EP 0 820 991, WO 94/12181, WO 94/08577, EP 0 518 586, WO 95/32710, WO 96/00574, WO 96/00730 oder in der DE 198 50 131 beschrieben.

Unter den MMP-Inhibitoren und den Tyrosin-Kinase-Inhibitoren sind die oben genannten bevorzugt.

### Beispiel zum Testen einer Kombinationstherapie:

Verzögerung des Tumorwachstums analog Kakeji (F. Mitjans et al., J. Cell Sci. 108: 2825-2838 (1995)):

Lewis-Lungenkarzinomzellen (2x 10E6) werden in 8-10 Wochen alte C57BL-Mäuse injiziert. Am vierten Tag wird Cyclo-(Arg-Gly-Asp-D-Phe-NMe-Val) (30 mg/kg) täglich i.p. verabreicht. Das Tumorwachstum wird täglich gemessen (B.A. Teicher et al., Int. J. Cancer 57: 920-925 (1994)). Haben die Tumoren ein bestimmtes Volumen von ungefähr 100 mm³ erreicht, beginnen am Tag 7 nach Tumorinoculation verschiedene cytotoxische Kombinationstherapien durch intraperitoneale Injektion. Beispiele: 5-Fluorouracil (30 mg/kg) oder Adriamycin (1,8 mg/kg) werden täglich von Tag 7 bis 11 gegeben. Cyclophosphamid (150 mg/kg), Carmustine (15 mg/kg) oder Gemcitabine (2,5 mg/kg) werden am Tag 7, 9 und 11 gegeben. Cisplatin (10 mg/kg) wird am Tag 7 gegeben.
Die Tumoren werden dreimal die Woche gemessen bis ein Volumen von ungefähr 500 mm³ erreicht ist. Die Verzögerung des Tumorwachstums wird als die Zeit berechnet, die ein individueller Tumor benötigt um 500 mm³ zu erreichen im Vergleich zu unbehandelten Kontrollen.

Die nachfolgenden Beispiele betreffen pharmazeutische Zubereitungen:

### Beispiel A: Injektionsgläser

Eine Lösung von 100 g Cyclo-(Arg-Gly-Asp-D-Phe-NMe-Val) und/oder eines seiner physiologisch unbedenklichen Salze, 100 g des Chemotherapeutikums und 5 g Dinatriumhydrogenphosphat wird in 6 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg der Wirkstoffe.

### Beispiel B: Suppositorien

Man schmilzt ein Gemisch von 20 g Cyclo-(Arg-Gly-Asp-D-Phe-NMe-Val) und/oder eines seiner physiologisch unbedenklichen Salze, 20 g des Chemotherapeutikums mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g Cyclo-(Arg-Gly-Asp-D-Phe-NMe-Val) und/oder eines seiner physiologisch unbedenklichen Salze, 1 g des Chemotherapeutikums, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg Cyclo-(Arg-Gly-Asp-D-Phe-NMe-Val) und/oder eines seiner physiologisch unbedenklichen Salze, 500 mg des Chemotherapeutikums mit 99,5 g Vaseline unter aseptischen Bedingungen.

### Beispiel E: Tabletten

Ein Gemisch von 1 kg Cyclo-(Arg-Gly-Asp-D-Phe-NMe-Val) und/oder eines seiner physiologisch unbedenklichen Salze, 1 kg des Chemotherapeutikums, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Cyclo-(Arg-Gly-Asp-D-Phe-NMe-Val) und/oder eines seiner physiologisch unbedenklichen Salze, 2 kg des Chemotherapeutikums werden in üblicher Weise in Hartgelatinekapsein gefüllt, so daß jede Kapsel 20 mg des Wirkstoffs enthält.

### Beispiel H: Ampullen

Eine Lösung von 1 kg Cyclo-(Arg-Gly-Asp-D-Phe-NMe-Val) und/oder eines seiner physiologisch unbedenklichen Salze, 1 kg des Chemotherapeutikums in 60 I zweifach destilliertem Wasser wird steril filtriert, in Ampullen abgefüllt, unter sterilen Bedingungen lyophilisiert und steril verschlossen. Jede Ampulle enthält je 10 mg der Wirkstoffe.

### Beispiel I: Set (Kit)

### Zubereitung (Kit) zur parenteralen Anwendung

Die Zubereitung enthält 500 mg Cyclo-(Arg-Gly-Asp-D-Phe-NMe-Val) und/oder eines seiner physiologisch unbedenklichen Salze und 500 mg Gemcitabine Hydrochlorid und wird wie folgt hergestellt:
Je 500 mg der beiden Verbindungen werden in 40 mL destillierten Wassers gelöst. Die Lösungen werden unter sterilen Bedingungen filtriert und in 10 ml Ampullen abgefüllt und lyophilisiert.
Zur intravenösen oder intramuskulären Injektion wird mit 10 mL 5%iger wässriger Dextrose versetzt.

## Patentansprüche

1. Pharmazeutische Zubereitung enthaltend Cyclo-(Arg-Gly-Asp-D-Phe-NMe-Val) und/oder eines seiner physiologisch unbedenklichen Salze und mindestens ein Chemotherapeutikum und/oder eines seiner physiologisch unbedenklichen Salze, dadurch gekennzeichet, dass das Chemotherapeutikum Gemcitabine eingesetzt wird.

2. Pharmazeutische Zubereitung nach Anspruch 1 enthaltend mindestens einen Angiogeneseinhibitor und/oder eines seiner physiologisch unbedenklichen Salze.

3. Pharmazeutische Zubereitung nach Anspruch 2, **dadurch gekennzeichnet, dass** ein Angiogeneseinhibitor aus einer Gruppe bestehend aus ανβ₃- und ανβ₅-Integrininhibitoren eingesetzt wird.

4. Pharmazeutische Zubereitung nach einem der Ansprüche 1 - 3, enthaltend mindestens einen MMP-Inhibitor und/oder eines seiner physiologisch unbedenklichen Salze.

5. Pharmazeutische Zubereitung nach einem der Ansprüche 1 - 4, enthaltend mindestens einen Tyrosin-Kinase-Inhibitor und/oder eines seiner physiologisch unbedenklichen Salze.

6. Verwendung einer pharmazeutischen Zubereitung gemäß den Ansprüchen 1 -5 zur Herstellung eines Arzneimittels zur Bekämpfung von Tumoren.

7. Verwendung von Cyclo-(Arg-Gly-Asp-D-Phe-NMe-Val) und/oder einem seiner physiologisch unbedenklichen Salze nacheinander oder in physikalischer Kombination mit Gemcitabine und/oder einem seiner physiologisch unbedenklichen Salze zur Herstellung eines Arzneimittels zur Bekämpfung von Tumoren.

8. Verwendung von Cyclo-(Arg-Gly-Asp-D-Phe-NMe-Val) und/oder einem seiner physiologisch unbedenklichen Salze nacheinander oder in physikalischer Kombination mit Gemcitabine und/oder einem seiner physiologisch unbedenklichen Salze und einem Angiogeneseinhibitor und/oder einem seiner physiologisch unbedenklichen Salze zur Herstellung eines Arzneimittels zur Bekämpfung von Tumoren.

9. Set, bestehend aus getrennten Packungen von
a) einer wirksamen Menge an Cyclo-(Arg-Gly-Asp-D-Phe-NMe-Val) und
b) einer wirksamen Menge des Chemotherapeutikums Gemcitabine.

## Claims

1. Pharmaceutical composition comprising cyclo(Arg-Gly-Asp-D-Phe-NMe-Val) and/or one of its physiologically acceptable salts and at least one chemotherapeutic agent and/or one of its physiologically acceptable salts, **characterised in that** the chemotherapeutic agent employed is gemcitabine.

2. Pharmaceutical composition according to Claim 1 comprising at least one angiogenesis inhibitor and/or one of its physiologically acceptable salts.

3. Pharmaceutical composition according to Claim 2, **characterised in that** an angiogenesis inhibitor from a group consisting of αvβ₃ and αvβ₅ integrin inhibitors is employed.

4. Pharmaceutical composition according to one of Claims 1 - 3, comprising at least one MMP inhibitor and/or one of its physiologically acceptable salts.

5. Pharmaceutical composition according to one of Claims 1 - 4, comprising at least one tyrosine kinase inhibitor and/or one of its physiologically acceptable salts.

6. Use of a pharmaceutical composition according to Claims 1-5 for the preparation of a medicament for combating tumours.

7. Use of cyclo(Arg-Gly-Asp-D-Phe-NMe-Val) and/or one of its physiologically acceptable salts one after the other or in physical combination with gemcitabine and/or one of its physiologically acceptable salts for the preparation of a medicament for combating tumours.

8. Use of cyclo(Arg-Gly-Asp-D-Phe-NMe-Val) and/or one of its physiologically acceptable salts one after the other or in physical combination with gemcitabine and/or one of its physiologically acceptable salts and an angiogenesis inhibitor and/or one of its physiologically acceptable salts for the preparation of a medicament for combating tumours.

9. Set consisting of separate packs of
a) an effective amount of cyclo(Arg-Gly-Asp-D-Phe-NMe-Val) and
b) an effective amount of the chemotherapeutic agent gemcitabine.

## Revendications

1. Composition pharmaceutique comprenant cyclo(Arg-Gly-Asp-D-Phe-NMe-Val) et/ou l'un de ses sels physiologiquement acceptables et au moins un agent chimiothérapeutique et/ou l'un de ses sels physiologiquement acceptables, **caractérisée en ce que** l'agent chimiothérapeutique utilisé est gemcitabine.

2. Composition pharmaceutique selon la revendication 1, comprenant au moins un inhibiteur d'angiogénèse et/ou l'un de ses sels physiologiquement acceptables.

3. Composition pharmaceutique selon la revendication 2, **caractérisée en ce qu'**un inhibiteur d'angiogénèse pris parmi un groupe constitué par des inhibiteurs d'intégrine αvβ₃ et αvβ₅ est utilisé.

4. Composition pharmaceutique selon l'une des revendications 1 à 3, comprenant au moins un inhibiteur de MMP et/ou l'un de ses sels physiologiquement acceptables.

5. Composition pharmaceutique selon l'une des revendications 1 à 4, comprenant au moins un inhibiteur de kinase tyrosine et/ou l'un de ses sels physiologiquement acceptables.

6. Utilisation d'une composition pharmaceutique selon les revendications 1 à 5 pour la préparation d'un médicament pour combattre les tumeurs.

7. Utilisation de cyclo(Arg-Gly-Asp-D-Phe-NMe-Val) et/ou de l'un de ses sels physiologiquement acceptables l'un après l'autre ou en combinaison physique avec gemcitabine et/ou l'un de ses sels physiologiquement acceptables pour la préparation d'un médicament pour combattre les tumeurs.

8. Utilisation de cyclo(Arg-Gly-Asp-D-Phe-NMe-Val) et/ou de l'un de ses sels physiologiquement acceptables l'un après l'autre ou en combinaison physique avec gemcitabine et/ou l'un de ses sels physiologiquement acceptables et d'un inhibiteur d'angiogénèse et/ou de l'un de ses sels physiologiquement acceptables pour la préparation d'un médicament pour combattre les tumeurs.

9. Ensemble constitué par des groupes séparés de
a) une quantité efficace de cyclo(Arg-Gly-Asp-D-Phe-NMe-Val) et
b) une quantité efficace de l'agent chimiothérapeutique gemcitabine.
